# EUROPEAN PATENT APPLICATION

(11) **EP 3 649 993 A1**
(43) Date of publication of application: **13.05.2020**
(21) Application number: 19207638.8
(22) Date of filing: 07.11.2019
(51) Int. Cl.: A61F 5/01

(54) **ADJUSTABLE ORTHOPAEDIC ORTHOSIS**

(30) Priority: 08.11.2018 NL 2021955
(71) Applicant: WE Design Beheer B.V., 6814 EN Arnhem (NL)
(72) Inventor: Engelshoven, Wouter Robin, 6815 AK Arnhem (NL)
(74) Representative: Nederlandsch Octrooibureau

(57) **Abstract**

An orthosis (1) for support of a patient's wrist, hand and/or fingers, comprising a substantially rigid wire frame (2) which is shaped and configured to directly engage a patient's wrist, hand and/or fingers in snug fashion for support thereof. The wire frame (2) comprises one or more longitudinally extendible and retractable wire frame adjusters (3) for loosening or tightening the wire frame (2), wherein each of the wire frame adjusters (3) comprises a first frame end section (4) and a second frame end section (5) in side-by-side engagement, wherein the first and second frame end sections (4,5) are laterally immovable whilst being longitudinally movable with respect to each other. Each of the wire frame adjusters (3) is positioned at a predetermined location with respect to the wire frame (2) for providing longitudinal extension and retraction thereof at the predetermined location.

## Description

### Field of the invention

The present invention relates to an adjustable orthopaedic orthosis, in particular a wire-based adjustable orthosis for support of a patient's wrist, hand and/or fingers.

### Background art

There are known orthoses that are made of a wire frame comprising one or more bent wires that snugly fit to a patient's wrist, hand and/or fingers. One of the advantages of a wire based orthosis is that it is waterproof and that it provides a low profile and sleek design such that the orthosis does not interfere with clothing and that it can be worn over longer periods of time.

Because wire-based orthoses tend to be substantially rigid to provide the desired orthopaedic support, some movements of a patient may be overly restricted by the orthoses even through such movements would be acceptable from an orthopaedic point of view.

### Summary of the invention

The present invention seeks to provide an improved orthosis for support of a patient's wrist hand and/or fingers, wherein the orthosis allows for increased freedom of movement without sacrificing orthopaedic support where it is needed as well as allowing adjustments to be made to the fit of the orthosis.

According to the present invention, an orthosis of the type defined in the preamble is provided comprising a substantially rigid wire frame which is shaped and configured to directly engage a patient's wrist, hand and/or fingers in snug fashion for support thereof, wherein the wire frame comprises one or more longitudinally extendible and retractable wire frame adjusters each of which comprises a first frame end section and a second frame end section in side-by-side engagement, wherein the first and second frame end sections are laterally immovable whilst being longitudinally movable with respect to each other, and wherein each of the wire frame adjusters is positioned at a predetermined location with respect to the wire frame for providing longitudinal extension and retraction thereof at the predetermined location.

According to the invention, the side-by-side arrangement of the first frame end section and the second frame end section allows minor adjustments such as lengthening and shortening of the substantially rigid wireframe at the predetermined location through motion of the patient. Here, the predetermined location is a location at which wire frame adjustments do not compromise orthopaedic support of the orthosis yet allow some flexure, i.e. lengthening and shortening, of the orthosis at the predetermined location.

In an advantageous embodiments the wire frame, the first frame end section and the second frame end section are each made of a metallic or metal alloy material, thereby providing a substantially rigid wire frame capable of providing support to a patients wrist, hand, and/or fingers. In a further advantageous embodiment, the wire frame, the first frame end section and the second frame end section each comprise silver, gold, bronze, titanium or a combination thereof, thereby providing antibacterial properties such that the orthosis can be worn for extended period of times and/or for performing tasks for which prior art orthoses would typically be temporarily removed, such as various household cleaning activities etc.

In an embodiment, the wire frame, and in particular the first and second frame end sections, comprise an oval shaped cross section (e.g. "flattened" cross section) thereby providing an enlarged surface area to the wire frame, and the first and second end frame sections, that comes into direct contact with a patient's skin, reducing pressure point and increasing comfort. The oval shaped or flattened cross section further reduces a thickness or height of the wire frame with respect to a patient wrist hand and/or fingers, providing a sleeker profile that minimizes interference with clothes.

In view of the oval shaped/flattened cross section of the wire frame, e.g. the wire frame adjusters, and in particular the side-by-side movable engagement between the first and second frame end sections, a thickness/height or width thereof can be optimized depending on orthopaedic needs of a patient.

For example, in an embodiment the first frame end section and the second frame end section are in side-by-side engagement along their widest side. In this embodiment, the first and second frame end sections may be viewed as being a stacked arrangement in which the widest sides (the flattened sides) are in engagement. This embodiment allows for a reduced width of the wire frame adjusters making reduced direct contact with a patient's skin if orthopedic requirements are such that the width of the wire frame adjusters is constraint.

In a further exemplary embodiment, the first frame end section and the second frame end section are in side-by-side engagement along a their narrowest side. In this embodiment the narrows side of the oval shaped/flattened cross section of the first and second frame end section are in engagement, allowing for a reduced thickness/height of the wire frame adjusters making more direct contact with a patient's skin if orthopedic requirements are such that the thickness/height of the wire frame adjusters is constraint.

### Short description of drawings

The present invention will be discussed in more detail below, with reference to the attached drawings, in which
Figure 1 shows a dorsal view of a hand orthosis provided with an adjustable wire frame according to an embodiment of the present invention.
Figure 2 shows a volar view of a hand orthosis provided with an adjustable wire frame to an embodiment of the present invention.
Figure 3 shows a dorsal view of a wrist orthosis provided with an adjustable wire frame according to an embodiment of the present invention;
Figure 4 shows an inner view of an adjustable wire frame according to an embodiment of the present invention;
Figure 5 shows an outer view of an adjustable wire frame according to an embodiment of the present invention;
Figure 6 shows an ulnar view of a wrist orthosis provided with an adjustable wire frame according to an embodiment of the present invention; and
Figure 7 shows a volar view of a wrist orthosis provided with an adjustable wire frame according to an embodiment of the present invention.

### Detailed description of embodiments

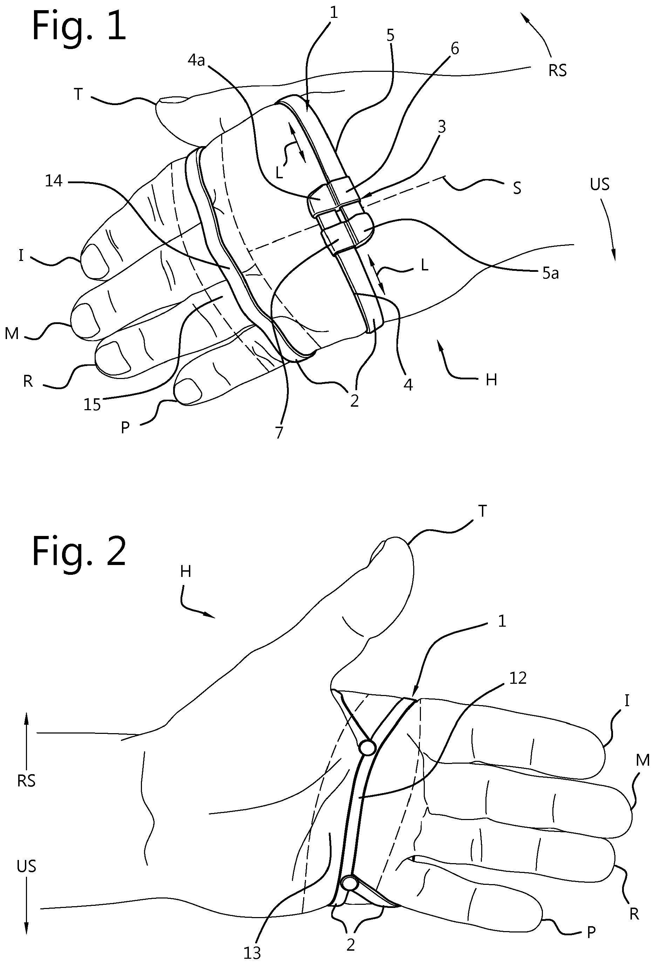

Figures 1 and 2 show a dorsal and volar view of an orthosis 1, e.g. a hand/finger orthosis 1, respectively, with an adjustable wire frame 2 according to an embodiment of the present invention. In the embodiment shown, the orthosis 1 comprises a substantially rigid wire frame 2 which is shaped, e.g. bent, and configured to directly engage a patient's wrist, hand and/or fingers in snug fashion for support thereof. That is, the wire frame 2 is shaped to be in direct skin contact with a patient.

The wire frame 2 comprises one or more longitudinally extendible and retractable wire frame adjusters 3 each of which comprise a first frame end section 4 and a second frame end section 5 in side-by-side engagement. Each of the first and second frame end sections 4, 5 may be viewed as representing a local end point or discontinuity of the wire frame 2. Further, the first and second frame end sections 4, 5 are immovable with respect to each in a lateral/sideways direction "S" whilst being movable with respect to each other in a longitudinal direction "L" as indicated in Figure 1. The longitudinal direction L may be considered to be a direction parallel to a direction in which the first and second frame end sections 4, 5 extend in lengthwise sense.

Based on orthopaedic needs, each of the wire frame adjusters 3 is positioned at a predetermined location with respect to the wire frame 2 for providing longitudinal extension and retraction of the wire frame 2 at the predetermined location.

According to the invention, the longitudinally moveable side-by-side engagement between the first frame end section 4 and the second frame end section 5 allows minor adjustments such as lengthening and shortening of the substantially rigid wire frame 2 at the predetermined location through e.g. motion of the patient's wrist, hand, and/or fingers. Also, the one or more wire frame adjusters 3 may even allow minor changes to the fit of the orthosis 1 by longitudinally moving the first and second frame end sections 4 up to a point where the wire frame 2 starts to bent and maintains its bent shape, thereby allowing minor permanent changes of the orthoses 1 if so needed.

In an embodiment, the wire frame 2 may be made of a metallic or metal alloy material such that a substantially rigid wire frame 2 can be obtained by bending it into shape for a snug direct engagement with a patient's skin. In an advantageous embodiment the wire frame 2 is made of silver, gold, bronze, titanium or a combination thereof, thereby providing anti-bacterial properties to the orthosis 1 such that the orthosis 1 can be worn over prolonged periods of time.

More specifically, in an embodiment the wire frame 2, the one or more wire frame adjusters 3 as well as the first and second frame end sections 4, 5 of the wire frame 2 may all be made of a metallic or metal alloy wire based material which can be bent/formed to obtain a required shape of the wire frame 2, the wire frame adjusters 3 and the first and second frame end sections 4, 5 thereof.

In a further embodiment the one or more wire frame adjusters 3 and the first and second frame end sections 4, 5 of the wire frame 2 may all be made of silver, gold, bronze, titanium or a combination thereof, thereby providing anti-bacterial properties to the orthosis 1, i.e. the entire wire frame 2 with the wire frame adjusters 3 and the first and second frame end sections 4, 5, such that the orthosis 1 can be worn safely over prolonged periods of time.

In an exemplary embodiment, the wire frame 2 including the one or more wire frame adjusters 3 and the first and second frame end sections 4, 5 thereof may all be made of 92,5% silver (purity 99.999) and 7,5% copper (purity 99.999), sometimes referred to as 925/1000 silver, for optimal rigidity versus flexibility and anti-bacterial properties. The orthosis 1 as shown in Figures 1 and 2 is a hand/finger orthosis for support of the metacarpophalangeal joints (MCP) joints, sometimes referred to as a "knuckle bender". This particular embodiment of the orthoses 1 is shaped and configured to prevent hyperextension of e.g. the second to fifth MCP joints. As shown, the orthosis 1 is arranged around a patients (H), wherein the letters T, I, M, R refer to the thumb, index, middle, ring and pinky finger. The depicted wire frame adjuster 3 is laterally arranged along the dorsal side of the hand to allow for a widening fit or narrowing fit, for example. That is, the indicated longitudinal direction L of the wire frame adjuster 3, for extension and contraction between the first and second frame end sections 4, 5, extends latterly with respect to the dorsal side of the hand. So even though the orthosis 1 comprises a substantially rigid frame 2, the wire frame adjuster 3 still allows for some freedom of adjustment of the orthosis to the patient's hand to improve comfort and to optimize the fit to a patient. Further details on the depicted orthosis in Figure 1 and 2 will be discussed later.

In an embodiment, the first frame end section 4 and the second frame end section 5 of the wire frame 2 each comprise an oval shaped or flattened cross section, thereby providing a larger surface area to make direct contact with a patient skin for reducing pressure points and improve comfort. Of course, the entire wire frame 2 including the first and second frame end sections 4, 5 may have an oval shaped or flattened cross section for enlarged skin contact hence improved comfort and support.

In an exemplary embodiment, the wire frame 2 and the first and second frame end sections 4, 5 thereof may be made of wire-based material having a width between 4 mm and 8 mm, preferably between 5 mm and 7mm, e.g. 6 mm. These widths allow for the aforementioned larger surface area to make direct contact with a patient skin for reducing pressure points and improve comfort.

In a further exemplary embodiment, the wire frame 2 and the first and second frame end sections 4, 5 thereof may be made of wire-based material having a thickness between 1 mm and 3 mm, e.g. 2 mm. These thicknesses prevent the wire frame 2 becoming too thick, possibly interfering with e.g. clothing.

Using the aforementioned wire specifications, such as 925/1000 silver and/or the widths (e.g. 4mm-8mm, 5mm-7mm, 6mm) and thicknesses (e.g. 1mm-3mm, 2mm) of the wire frame 2 including the first and second frame end sections 4, 5, allows for a sleek and low profile orthosis 1. Also, these wire specifications provide sufficient mechanical rigidity for orthopedic support whilst allowing the wire frame 2 to be shaped/bent for achieving an excellent fit of the orthosis 1 to the patient.

It is worth noting that in further embodiments it is conceivable that the entire wire frame 2 of the orthosis 1 need not be made of wire-based material having the same width and thickness everywhere. For example, the first and second frame end sections 4, 5 may have a different width and/or thickness than remaining parts of the wire frame 2. Likewise, it is conceivable that the wire frame 2 changes in width and/or thickness for different areas of the wrist, hand and/or fingers to be engaged by the wire frame 2.

In a further embodiment, the first frame end section 4 and the second frame end section 5 are in side-by-side engagement along their narrowest side. In this embodiment the narrows side of the oval shaped or flattened cross section of the first and second frame end sections 4, 5 are in longitudinally movable engagement, allowing for a reduced thickness/height of a wire frame adjuster 3 making enlarged direct contact with a patient's skin if orthopedic requirements are such that the thickness/height of the wire frame adjusters is a constraint or poses other problems. This exemplary embodiment is depicted in Figure 1, wherein the wire frame adjuster 3 comprises the first and second wire frame end seconds 4, 5 having an oval shaped or flattened cross section, e.g. being made of flat wire material. Because the narrowest side of the first and second wire frame end seconds 4, 5 are in engagement provides a large skin surface contact to the wire frame adjuster 3 increasing comfort, such as at the dorsal side of the hand with thin tissue at which painful pressure points may develop quickly.

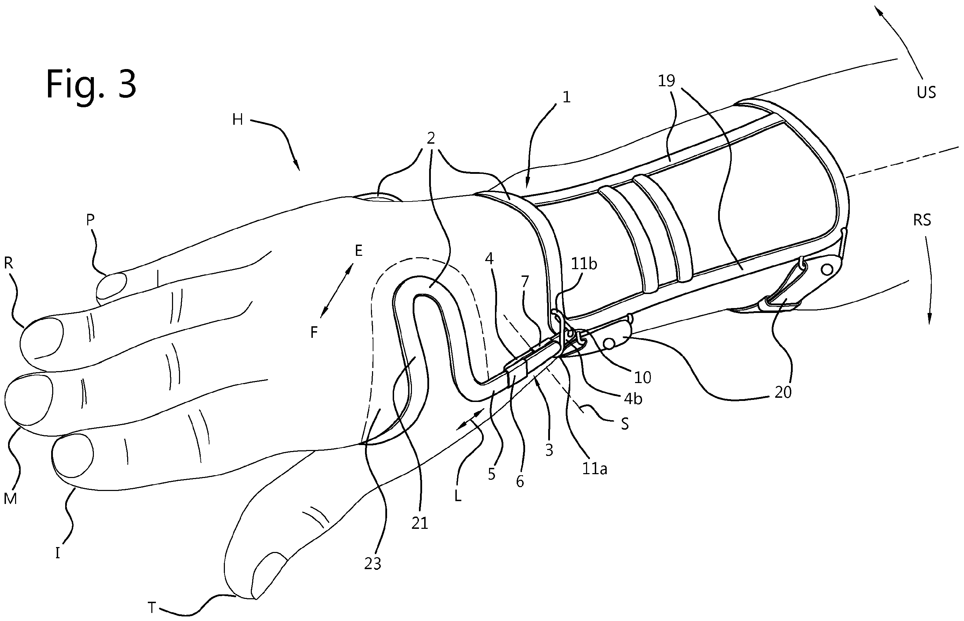

Figure 3 shows a dorsal view of another orthosis 1, e.g. a wrist orthosis 1, provided with an adjustable wire frame 3 according to an exemplary embodiment of the present invention. In this embodiment shown, the orthosis 1 comprises the substantially rigid wire frame 2 which is shaped, e.g. bent, and configured to directly engage a patient's wrist and hand in snug fashion for support thereof. The wire frame 2 comprises the one or more longitudinally extendible and retractable wire frame adjusters 3 each of which comprises the first frame end section 4 and a second frame end section 5 in side-by-side engagement. Each of the first and second frame end sections 4, 5 may be viewed as representing a local end point or discontinuity of the wire frame 2.

The first and second frame end sections 4, 5 are immovable with respect to each in the lateral/sideways direction "S" whilst being movable with respect to each other in a longitudinal direction "L" as indicated in Figure 3. The longitudinal direction L may be considered to be a direction parallel to a direction in which the first and second frame end sections 4, 5 move in relative fashion in lengthwise sense. Based on orthopaedic needs, each of the wire frame adjusters 3 is positioned at a predetermined location with respect to the wire frame 2 for providing longitudinal extension and retraction of the wire frame 2 at the predetermined location. In the exemplary embodiment depicted, the predetermined location for the wire frame adjuster 3 is somewhere along the radial side RS of the wrist area. Although not shown, in a further embodiment another wire frame adjuster 3 may be arranged at a ulnar side US of the wrist area. Further details of the depicted wrist orthosis will be discussed later.

For maximum clarity, the term "ulnar side" is associated with a side indicated by the direction of the arrow labelled "US", and wherein the term "radial side" is associated with a side indicated by the direction of the arrow labelled "RS".

Like the orthosis 1 shown in Figure 1, in Figure 3 an embodiment is depicted wherein the first frame end section 4 and the second frame end section 5 of the wire frame 2 each comprise the oval shaped or flattened cross section, such as flat wire material, to increase skin surface contact and as such provide more comfort to a patient by minimizing pressure points.

In a further embodiment, as shown in Figure 3, the first frame end section 4 and the second frame end section 5 are in (longitudinally movable) side-by-side engagement along their widest side. That is, in this embodiment a widest side of the oval shaped or flattened cross section of each of the first and second frame end sections 4, 5 are in engagement, providing a stacked arrangement of the first and second frame end sections 4, 5 with respect to the patients skin. As shown in Figure 3, by providing a side-by-side engagement between the first and second frame end section 3 along their widest side, the wire frame adjuster 3 becomes less wide and exhibits reduced skin contact should this be desired from an orthopaedic point of view.

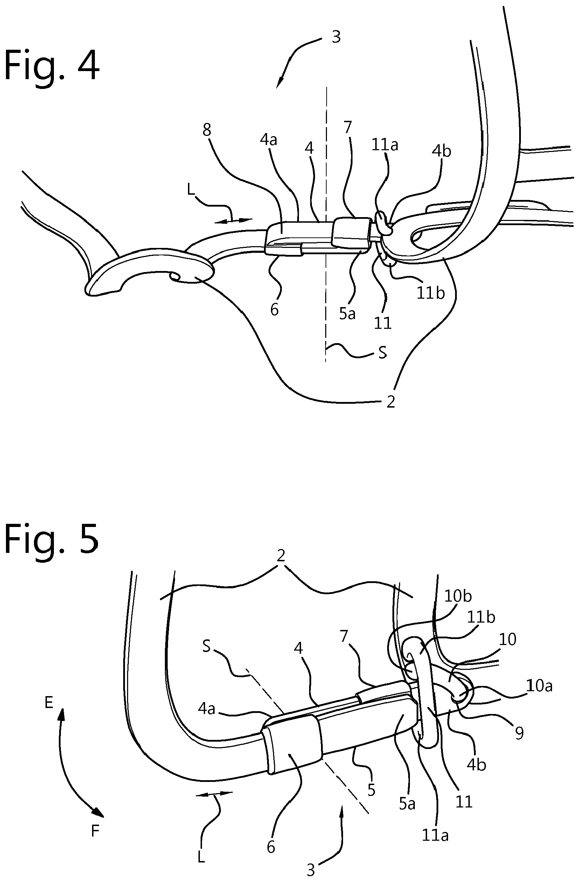

Referring to Figures 4 and 5 each showing an inner and outer view, respectively, of a wire frame adjuster 3 according to an embodiment of the present invention. In an embodiment as shown, the first frame end section 4 may comprises a frame eyelet 6, i.e. a first frame eyelet 6, at a frame end point 4a of the first frame end section 4, and wherein the second frame end section 5 movably extends, e.g. in the longitudinal direction L, through the frame eyelet 6, i.e. the first frame eyelet 6. In this particular embodiment, the first frame eyelet 6 may be seen as being wrapped around the second frame end section 5, thereby restricting/preventing lateral movement in the lateral direction S but allowing longitudinal movement, e.g. through sliding, in the longitudinal direction L between the first and second frame end section 4, 5.

To further increase lateral stability of the wire frame adjuster 3, an embodiment is provided wherein the second frame end section 5 comprises a further frame eyelet 7, i.e. a second frame eyelet 7, at a frame end point 5a of the second frame end section 5, wherein the first frame end section 4 moveably extends through the second frame eyelet 7. Also in this particular embodiment the second frame eyelet 6 may be seen as being wrapped around the first frame end section 4 to restrict/prevent lateral movement in the lateral direction S but to allow longitudinal movement L ,e.g. through sliding, between the first and second frame end sections 4, 5.

In an advantageous embodiment, the first and/or second frame eyelets 6, 7 may each be made of the same material as the wire frame 2, i.e. the same material as the first and second frame end sections 4, 5, thereby providing substantial rigidity to the first and/or second frame eyelets 6, 7, hence lateral stability and durability of the wire frame adjuster 3.

In a further advantageous embodiment, the first and/or second frame eyelets 6, 7 may each be made of a metallic or metal alloy material to provide the sufficient rigidity and durability as mentioned earlier. Moreover, having the first and/or second frame eyelets 6, 7 of a metallic or metallic alloy material allows the wire frame adjuster 3 to be fixed into a particular position by pressing the first and/or second eyelet 6, 7 with e.g. pliers. This may be advantageous as the orthosis 1 may be made to fit properly by adjustment in situ by the wire frame adjuster 3, i.e. when the patient actually wears the orthosis 1 and by using the wire frame adjuster 3 and its longitudinal extendibility and retractability to achieve a perfect fit by pressing/pinching the first and/or second frame eyelets 6, 7 for affixing a particular position between the first and second frame end sections 4, 5.

As with the wire frame 2 and the first and second frame end sections 4, 5 thereof, in an embodiment the first and/or second eyelets 6, 7 may be made of silver, gold, bronze, titanium or a combination thereof, which further increases durability of the orthosis 1 and reduces bacterial development, e.g. between the orthosis 1 and a patients skin, and as such allows the orthosis 1 to be worn over longer periods of time.

As depicted in Figure 4, an embodiment may be provided wherein the frame end point 4a of the first frame end section 4 comprises a skin engaging tapered edge 8, thereby preventing a sharp edge on the frame end point 4a when it comes into contact with a patient's skin, avoiding pressure points. In a particular embodiment, when the first frame end section 4 and frame end point 4a thereof have an oval shaped or flattened cross section, the tapered edge 8 may be provided on a skin engaging widest side of the frame end point 4a, so that there is smooth tapered transition from a skin engaging widest side of the first frame end section 4 toward the frame end point 4a.

From an orthopedic point of view, allowing adjustability of the substantially rigid wire frame 2 in the depicted longitudinal direction L through one or more wire frame adjusters is very valuable for obtaining a personalized fit of the orthosis 1. As the one or more wire frame adjusters 3 allows for longitudinal extension and retraction of the wire frame 2 at the predetermined location, there are scenarios conceivable wherein the orthosis 1, and in particular the wire frame 2, should allow for increased rotational movement when required.

For example, as depicted in Figure 5, the first frame end section 4 may be pivotally connected to the wire frame 2 at a pivot point 4b of the first frame end section 4. This embodiment allows the wire frame adjuster 3 to rotate at the pivot point 4b to increase freedom of movement of the orthosis 1 and hence a patient. For example, the orthosis 1 as shown in Figure 3, e.g. a wrist orthosis, may allow at least to some flexion or extension of the hand insofar allowable from an orthopaedic point of view. By having the first frame end section 4 pivotally connected the wire frame 2 by means of the pivot point 4b, allows rotational movement in addition to the longitudinal extension and retraction of the wire frame 2 by the wire frame adjuster 3.

In an exemplary embodiment, the pivot point 4b of the first frame end section 4 may comprise a pivot hole 9 and wherein the wire frame 2 comprises a primary pivot frame portion 10 affixed to the wire frame 2 and wherein the primary pivot frame portion 10 extends through the pivot hole 7, yielding a simple yet reliable pivotable connection between the wire frame 2 and the first frame end section 4.

As further depicted in Figure 5, in an embodiment the primary pivot frame portion 10 may be an arched frame portion having two opposing ends 10a, 10b each of which is affixed to the wire frame 2. This embodiment readily allows for wire based material to be used and bent to obtain the primary pivot frame portion 10 and wherein the opposing ends 10a, 10b thereof may be welded or soldered to the wire frame 2.
In an advantageous embodiment, the primary pivot frame portion 10 may be made of a metallic or metal alloy material, providing rigidity and durability. Moreover, in yet a further advantageous embodiment the primary pivot frame portion 10 may comprise gold, bronze, titanium or a combination thereof, adding further antibacterial properties to the primary pivot portion 10 as dirt may accumulate in the pivot hole 9 and on the primary pivot portion 10.

As mentioned earlier, the orthosis 1 of the present invention may further provide rotational freedom where allowable. However, in many cases the rotational freedom may need to be limited to still provide sufficient orthopaedic support and to prevent excessive movement.

For example, with reference to Figure 3, showing an exemplary embodiment of the orthosis 1 which may be configured to limit flexion "F" of the hand whilst allowing extension "E" in the direction as indicated. To that end an embodiment is provided wherein the wire frame 2 further comprises an arched secondary pivot frame portion 11 having two opposing ends 11a, 11b each being affixed to the wire frame 2, and wherein the first frame end section 4 extends underneath or through the arched secondary frame pivot portion 11. In this embodiment, the first frame end section 4 is pivotally movable by virtue of the pivot point 4b thereof, but wherein the arched secondary frame pivot portion 11 is configured to limit the rotational movement so that the first frame end section 4 is rotationally limited in the flexion direction "F" and the extension direction "E". Depending on the actual placement of the arched secondary frame pivot portion 11, the range of rotational displacement in the flexion direction F may be more limited than the range of rotational displacement in the extension direction E or vice versa.

For example, the orthosis 1 of Figure 3 shows an embodiment wherein the pivot point 4b may be arranged closer to a first end point 11a of the arched secondary frame pivot portion 11 than a second end point 11b thereof. Here, the first frame end section 4 extends through/underneath the arched secondary frame pivot portion 11, and wherein for some flexion angle, the first frame end section 4 abuts the first end point 11a of the arched secondary frame pivot portion 11, thereby preventing any further flexion to provide support for e.g. the hand/wrist. Since the second end point 11b is further away from the pivot point 4b, the orthosis 1 allows for a higher degree of rotation in the extension direction E. As is clear from Figure 5, placing the pivot point 4b closer to the second end point 11b of the arched secondary frame pivot portion 11 would limit rotation in the extension direction E and allow more rotation in the flexion direction F.

In an embodiment, the second frame eyelet 7 is longitudinally movable between the arched secondary pivot frame portion 11 and the first frame eyelet 6, thereby providing a limit to contraction and extension of the wire frame adjuster 3 to satisfy orthopaedic requirements.

In an embodiment, note that from Figure 1 it can be seen that the frame end point 5a of the second frame end section 5 may have a raised portion, so that extension of the wire frame adjuster 3 is limited through abutment of the first frame eyelet 6 with the raised portion of the frame end point 5a of the second frame end section 5. Likewise, in another embodiment the frame end point 4a of the first frame end section 4 may comprise a raised portion, so that extension of the wire frame adjuster 3 is limited through abutment of the second frame eyelet 7 with the raised portion of the frame end point 4a of the first frame end section 4.

In an embodiment, as depicted in Figure 1, the raised portion of the end point 4a of the first frame end section 4 may be part of the first frame eyelet 6. Likewise, in an embodiment the raised portion of the end point 5a of the second frame end section 5 may be part of the second frame eyelet 7. Therefore, both the first and/or second frame eyelet 6, 7 may be configured to provide a maximum extension to the wire frame adjuster 3.

From Figure 4 and 5 it is further seen that when the first frame end section 4 and the second frame end section 5 both have an oval shaped or flattened cross section and are in side-by-side engagement along their widest side, then the first frame eyelet 6 and/or the second frame may extend between the first and second frame end sections 4, 5 for providing a raised section as outlined earlier with which the extension of the wire frame adjuster 3 may be limited.

To further elaborate the usefulness of the wire frame adjuster 3 of the orthosis 1 to particular types of orthoses, reference is made to Figure 1 and 2, depicting an orthosis 1 for hand/finger support as mentioned earlier, e.g. a hand/finger orthosis. In the embodiments shown, the wire frame 2 may comprise a palmer wire section 12 configured to laterally extend across a palmar region 13 of the hand and a proximal phalanx wire section 14 configured to laterally extend along a proximal phalanx region 15 on a dorsal side of the proximal phalanx bones of the index I, middle M, ring R and pinky P finger, and wherein the wire frame 2 further comprises a single wire frame adjuster 3 for which the predetermined location is at a dorsal side of the hand, e.g. along a dorsal side of the metacarpal bones. This embodiment of the orthosis prevents hyperextension of the index I, middle M, ring R, and pinky P finger through the proximal phalanx wire section 14 and wherein the wire frame adjuster 3 allows specific adjustment to the orthosis 1 around the hand to obtain a snug fit.

Note that in an embodiment the palmer wire section 12 and the proximal phalanx wire section 14 of the wire frame 2 may all be made of a metallic or metal alloy wire based material which can be bent/formed to obtain the wire frame 2 in a specified form required for the patient.

In a further embodiment the palmer wire section 12 and the proximal phalanx wire section 14 of the wire frame 2 may all be made of silver, gold, bronze, titanium or a combination thereof, thereby providing anti-bacterial properties to the orthosis 1, i.e. the wire frame 2 including the wire frame adjusters 3, the first and second frame end sections 4, 5 as well as the palmer wire section 12 and the proximal phalanx wire section 14.

In an exemplary embodiment, the palmer wire section 12 and the proximal phalanx wire section 14 of the wire frame 2 may all be made of 92,5% silver (purity 99.999) and 7,5% copper (purity 99.999), sometimes referred to as 925/1000 silver, for reasons mentioned earlier.

Because it may be difficult to estimate a proper fit of the hand/wrist orthosis 1 around someone's hand, the wire frame adjuster 3 further allows in situ tightening or loosening of the wire frame 2 to obtain a perfect fit of the palmar wire section 12 toward the first and second frame end sections 4, 5. In an embodiment, by utilizing the first and second frame eyelets 6, 7 it is possible to fixate the wire frame adjuster 3 by pressing/squeezing the first and/or second frame eyelets 6, 7 once an appropriate fit has been found.

Referring to the embodiment of the orthosis 1 as shown in Figure 3, the depicted orthosis 1 may be viewed as a wrist orthosis 1 which, in this specific embodiment, provides support to the hand for preventing hyperflexion thereof.

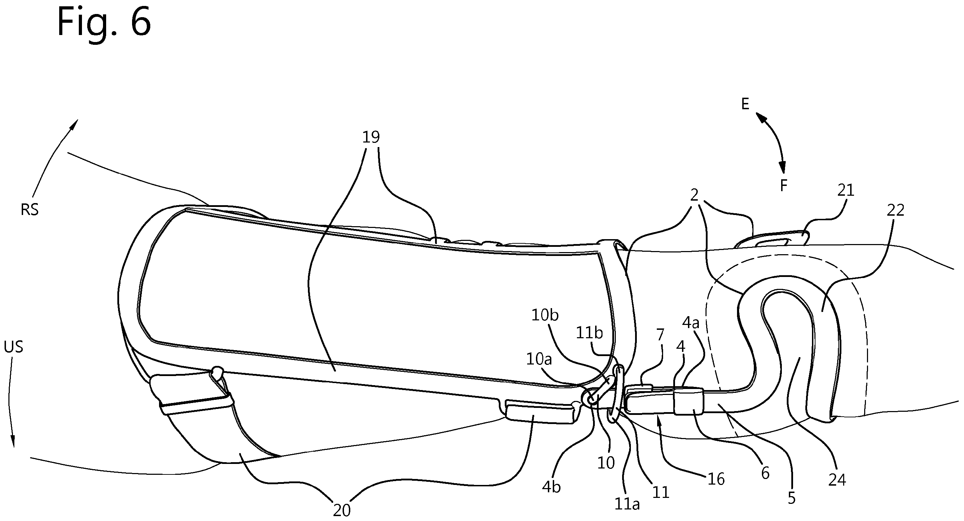

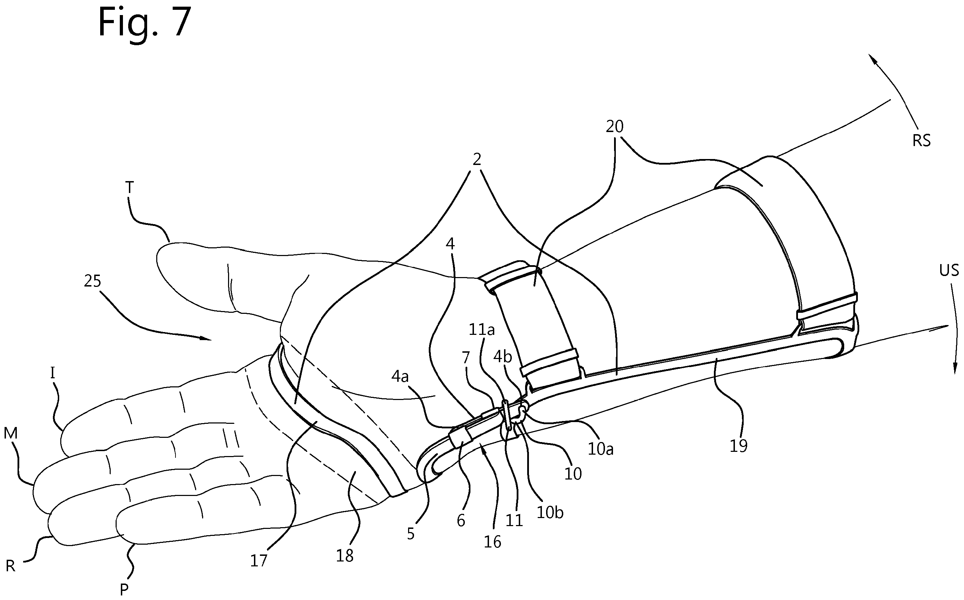

According to further embodiments of the present invention, Figures 6 and 7 each show an ulnar view and a volar view, respectively, of a wrist orthosis 1.

As shown, the wire frame 2 comprises two wire frame adjusters 3, 16, a palmer wire section 17 configured to laterally extend across a palmar region 18 of a hand for support thereof, and a forearm wire section 19 releasably attachable to a patient's forearm. The palmer wire section 17 and the forearm wire section 19 are connected through a first wire frame adjuster 3 along a radial side of a patient's wrist and/or hand and through a second wire frame adjuster 16 along an ulnar side of the patient's wrist and/or hand. In this embodiment the palmer wire section 17 provides support of the hand and wrist, such as limiting flexion thereof. Each of the wire frame adjusters 3, 16 allow a distance between the palmar wire section 12 and the forearm wire section 19 to be adjusted through extension or contraction of the two wire frame adjusters 3, 16, i.e. by moving the first and second frame end sections 4, 5 of each of the first and second wire frame adjusters 3, 16.

Note that in an embodiment the palmer wire section 17 and the forearm wire section 19 of the wire frame 2 may all be made of a metallic or metal alloy wire based material which can be bent/formed to obtain the wire frame 2 in a specified form required for the patient.

In a further embodiment the palmer wire section 17 and the forearm wire section 19 of the wire frame 2 may all be made of silver, gold, bronze, titanium or a combination thereof, thereby providing anti-bacterial properties to the orthosis 1, i.e. the wire frame 2 including the wire frame adjusters 3, the first and second frame end sections 4, 5 as well as the palmer wire section 17 and the forearm wire section 19.

In an exemplary embodiment, the palmer wire section 17 and the forearm wire section 19 of the wire frame 2 may all be made of 92,5% silver (purity 99.999) and 7,5% copper (purity 99.999), sometimes referred to as 925/1000 silver, for reasons mentioned earlier.

The two wire frame adjusters 3, 16 of the wrist orthosis 1 as shown in Figures 3, 6 and 7 further allow for radial deviation, i.e. rotation of the hand toward the radial side RS, and ulnar deviation, i.e. rotation of the hand toward the ulnar side US. For example, under radial deviation the first wire frame adjuster 3 may contract its first and second wire frame end sections 4, 5, and wherein the second wire frame adjuster 16 may extend its first and second wire frame end sections 4, 5. Under ulnar deviation the opposite occurs, i.e. contraction of the second wire frame adjuster 16 and extension of the first wire frame adjuster 3. Therefore, mutually opposing contraction and extension of the two wire frame adjusters 3, 16 allows for radial and ulnar deviation hence providing more freedom of movement to a patient when orthopedically allowed.

In case radial and ulnar deviation should not be allowed, then it is possible to utilize first and second frame eyelets 6, 7 of each of the two wire frame adjusters 3, 16 and prevent any contraction and extension thereof by pressing/clamping the first and/or second frame eyelet 6, 7. For example, pressing the first and/or second frame eyelet 6,7 would prevent longitudinal movement between the first and second wire frame end sections 4, 5 so that the wire frame adjusters 3, 16 are immobilized.

In an embodiment each of the two wire frame adjusters 3, 16 may be pivotally connected to the wire frame 2, e.g. through a pivot point 4b of the first frame end section 4 as explained earlier in light of Figures 4 and 5. To limit rotation in the flexion direction F and/or extension direction E as indicated in Figure 5 and 6, the wire frame 2 may comprise an arched secondary pivot frame portion 11 arranged on both a radial side RS and ulnar side US of the wire frame 2, i.e. near the wrist, wherein each arched secondary pivot frame portion 11 comprises two opposing ends 11a, 11b affixed to the wire frame 2, and wherein the first frame end section 4 of each wire frame adjuster 3, 16 extends through or underneath the arched secondary frame pivot portion 11. As a result, the arched secondary pivot frame portion 11 acts as an abutment for the first frame end section 4, limiting flexion as well as extension. For example, by choosing a distance between the pivot point 4b closer to the first end point 11a than the second end point 11b of the two opposing ends 11a, 11b, flexion of the hand can be further limited than extension of the hand. Conversely, by choosing a distance between the pivot point 4b closer to the second end point 11b than the first end point 11a of the two opposing ends 11a, 11b, extension of the hand can be further limited than flexion of the hand.

As further depicted in Figure 3 and 6, in an embodiment the palmar wire section 17 may be configured to extend around a radial side RS and ulnar side US of the hand and comprise a radial U-shaped bend 21 and an ulnar U-shaped bend 22 respectively. The radial U-shaped bend 21 is configured to support a (dorsal) radial region 23 of the hand and wherein the ulnar U-shaped bend 22 is configured to support a (dorsal) ulnar region 24 of the hand, thereby providing lateral support and stability and improved fit of the hand to the orthosis 1. Note that he palmar wire section 17 may be configured to extend around a radial side RS of the hand by extending through an interdigital space 25 between the thumb and index finger as shown.

In view of the above detailed description, the present invention can now be summarized clearly and concisely by the following embodiments:
Embodiment 1. An orthosis (1) for support of a patient's wrist, hand and/or fingers, comprising a substantially rigid wire frame (2) which is shaped and configured to directly engage a patient's wrist, hand and/or fingers in snug fashion for support thereof, wherein the wire frame (2) comprises one or more longitudinally extendible and retractable wire frame adjusters (3) for loosening or tightening the wire frame (2), wherein each of the wire frame adjusters (3) comprises
   a first frame end section (4) and a second frame end section (5) in side-by-side engagement, wherein the first and second frame end sections (4,5) are local end points of the wire frame (2), wherein the first and second frame end sections (4,5) are laterally immovable whilst being longitudinally movable with respect to each other, and wherein each of the wire frame adjusters (3) is positioned at a predetermined location with respect to the wire frame (2) for providing longitudinal extension and retraction of the wire frame (2) at the predetermined location.
Embodiment 2. The orthosis according to embodiment 1, wherein the first frame end section (4) and the second frame end section (5) of the wire frame (2) each comprise an oval shaped cross section.
Embodiment 3. The orthosis according to embodiment 2, wherein the first frame end section (4) and the second frame end section (5) are in side-by-side engagement along their widest side.
Embodiment 4. The orthosis according to embodiment 2, wherein the first frame end section (4) and the second frame end section (5) are in side-by-side engagement along their narrowest side.
Embodiment 5. The orthosis according to any of embodiments 1-4, wherein the first frame end section (4) comprises a first frame eyelet (6) at a frame end point (4a) of the first frame end section (4), and wherein the second frame end section (5) movably extends through the first frame eyelet (6).
Embodiment 6. The orthosis according to any of embodiments 1-5, wherein the second frame end section (5) comprises a second frame eyelet (7) at a frame end point (5a) of the second frame end section (5), wherein the first frame end section (4) moveably extends through the second frame eyelet (7).
Embodiment 7. The orthosis according to embodiment 5, wherein the frame end point (4a) of the first frame end section (4) comprises a skin engaging tapered edge (8).
Embodiment 8. The orthosis according to any of embodiments 1-7, wherein the first frame end section (4) is pivotally connected to the wire frame (2) at a pivot point (4b) of the first frame end section (4).
Embodiment 9. The orthosis according to embodiment 8, wherein the pivot point (4b) comprises a pivot hole (9) and wherein the wire frame (2) comprises a primary pivot frame portion (10) affixed to the wire frame (2) and extending through the pivot hole (9).
Embodiment 10. The orthosis according to embodiment 9, wherein the primary pivot frame portion (10) is an arched portion having two opposing ends (10a, 10b) each being affixed to the wire frame (2).
Embodiment 11. The orthosis according to embodiments 8, 9 or 10 wherein the wire frame (2) further comprises an arched secondary pivot frame portion (11) having two opposing ends (11a, 11b) each being affixed to the wire frame (2), wherein the first frame end section (4) extends underneath the arched secondary pivot frame portion (11).
Embodiment 12. The orthosis according to embodiment 11, when dependent on embodiment 5 and 6, wherein the second frame eyelet (7) is longitudinally movable between the arched secondary pivot frame portion (11) and the first frame eyelet (6).
Embodiment 13. The orthosis according to any of embodiments 1-6, wherein the wire frame (2) comprises a palmer wire section (12) configured to laterally extend across a palmar region (13) of the hand and a proximal phalanx wire section (14) which is configured to laterally extend along a proximal phalanx region (15) on a dorsal side of the proximal phalanx bones of the index (I), middle (M), ring (R) and pinky (P) finger, and wherein the wire frame (2) comprises one wire frame adjuster (3) for which the predetermined location is at a dorsal side of the hand.
Embodiment 14. The orthosis according to any of embodiments 1-12, wherein the wire frame (2) comprises two wire frame adjusters (3, 16),
   a palmer wire section (17) configured to laterally extend across a palmar region (18) of a hand for support thereof, and
   a forearm wire section (19) releasably attachable to a patient's forearm, and
   wherein the palmer wire section (17) and the forearm wire section (19) are configured to connect through a first wire frame adjuster (3) along a radial side of a patient's wrist and/or hand and through a second wire frame adjuster (16) along an ulnar side of the patient's wrist and/or hand.
Embodiment 15. The orthosis according to any of embodiments 1-14, wherein the wire frame (2), the first frame end section (4) and the second frame end section (5) are each made of a metallic or metal alloy material.
Embodiment 16. The orthosis according to any of embodiments 1-15, wherein the wire frame (2), the first frame end section (4) and the second frame end section (5) each comprise silver, gold, bronze, titanium or a combination thereof.

The present invention has been described above with reference to a number of exemplary embodiments as shown in the drawings. Modifications and alternative implementations of some parts or elements are possible, and are included in the scope of protection as defined in the appended claims.

## Claims

1. An orthosis (1) for support of a patient's wrist, hand and/or fingers, comprising a substantially rigid wire frame (2) which is shaped and configured to directly engage a patient's wrist, hand and/or fingers in snug fashion for support thereof, wherein the wire frame (2) comprises one or more longitudinally extendible and retractable wire frame adjusters (3) for loosening or tightening the wire frame (2), wherein each of the wire frame adjusters (3) comprises
a first frame end section (4) and a second frame end section (5) in side-by-side engagement, wherein the first and second frame end sections (4,5) are local end points of the wire frame (2), wherein the first and second frame end sections (4,5) are laterally immovable whilst being longitudinally movable with respect to each other, and wherein each of the wire frame adjusters (3) is positioned at a predetermined location with respect to the wire frame (2) for providing longitudinal extension and retraction of the wire frame (2) at the predetermined location.

2. The orthosis according to claim 1, wherein the first frame end section (4) and the second frame end section (5) of the wire frame (2) each comprise an oval shaped cross section.

3. The orthosis according to claim 2, wherein the first frame end section (4) and the second frame end section (5) are in side-by-side engagement along their widest side.

4. The orthosis according to claim 2, wherein the first frame end section (4) and the second frame end section (5) are in side-by-side engagement along their narrowest side.

5. The orthosis according to any of claims 1-4, wherein the first frame end section (4) comprises a first frame eyelet (6) at a frame end point (4a) of the first frame end section (4), and wherein the second frame end section (5) movably extends through the first frame eyelet (6).

6. The orthosis according to any of claims 1-5, wherein the second frame end section (5) comprises a second frame eyelet (7) at a frame end point (5a) of the second frame end section (5), wherein the first frame end section (4) moveably extends through the second frame eyelet (7).

7. The orthosis according to claim 5, wherein the frame end point (4a) of the first frame end section (4) comprises a skin engaging tapered edge (8).

8. The orthosis according to any of claims 1-7, wherein the first frame end section (4) is pivotally connected to the wire frame (2) at a pivot point (4b) of the first frame end section (4).

9. The orthosis according to claim 8, wherein the pivot point (4b) comprises a pivot hole (9) and wherein the wire frame (2) comprises a primary pivot frame portion (10) affixed to the wire frame (2) and extending through the pivot hole (9).

10. The orthosis according to claim 9, wherein the primary pivot frame portion (10) is an arched portion having two opposing ends (10a, 10b) each being affixed to the wire frame (2).

11. The orthosis according to claims 8, 9 or 10, wherein the wire frame (2) further comprises an arched secondary pivot frame portion (11) having two opposing ends (11a, 11b) each being affixed to the wire frame (2), wherein the first frame end section (4) extends underneath the arched secondary pivot frame portion (11).

12. The orthosis according to claim 11, when depending from claim 5 and 6, wherein the second frame eyelet (7) is longitudinally movable between the arched secondary pivot frame portion (11) and the first frame eyelet (6).

13. The orthosis according to any of claims 1-6, wherein the wire frame (2) comprises a palmer wire section (12) configured to laterally extend across a palmar region (13) of the hand and a proximal phalanx wire section (14) which is configured to laterally extend along a proximal phalanx region (15) on a dorsal side of the proximal phalanx bones of the index (I), middle (M), ring (R) and pinky (P) finger, and wherein the wire frame (2) comprises one wire frame adjuster (3) for which the predetermined location is at a dorsal side of the hand.

14. The orthosis according to any of claims 1-12, wherein the wire frame (2) comprises two wire frame adjusters (3, 16),
a palmer wire section (17) configured to laterally extend across a palmar region (18) of a hand for support thereof, and
a forearm wire section (19) releasably attachable to a patient's forearm, and
wherein the palmer wire section (17) and the forearm wire section (19) are configured to connect through a first wire frame adjuster (3) along a radial side of a patient's wrist and/or hand and through a second wire frame adjuster (16) along an ulnar side of the patient's wrist and/or hand.

15. The orthosis according to any of claims 1-14, wherein the wire frame (2), the first frame end section (4) and the second frame end section (5) each comprise silver, gold, bronze, titanium or a combination thereof.
